# EUROPEAN PATENT SPECIFICATION

(11) **EP 0 680 964 B1**
(45) Date of publication and mention of the grant of the patent: **16.01.2002**
(21) Application number: 95900773.3
(22) Date of filing: 18.11.1994
(51) Int. Cl.: C07D 487/04, C07D 487/10, A61K 31/40

(54) **PYRROLO [3,2-e]INDOLE DERIVATIVES, PROCESS FOR THE PREPARATION THEREOF AND THEIR USE**
PYRROLO [3,2-e]INDOLDERIVATE, VERFAHREN ZU IHRER HERSTELLUNG UND IHRE VERWENDUNG
DERIVES DE PYRROLO [3,2-e]INDOLE, PROCEDE DE PREPARATION ET LEUR UTILISATION

(30) Priority: 19.11.1993 ES 9300243
(43) Date of publication of application: 08.11.1995
(73) Proprietor: UNIVERSIDAD DE SANTIAGO DE COMPOSTELA, E-15701 Santiago de Compostela (ES)
(72) Inventor: DELAMANO GARCIA, José, E-36619 Villagarcia de Arosa (ES); TOJO SUAREZ, Gabriel, E-Santiago de Compostela (ES); LOPEZ GOTI, Carmen, E-15579 De Jubia Nede (ES); FERNANDEZ ALMEIDA, Jesús, E-49001 Zamora (ES); GARCIA GRAVALOS, Dolores, E-28006 Madrid (ES); FAIRCLOTH, Glynn Thomas, 10 Rogers Street, Cambridge, MA 02142 (US)
(74) Representative: Ungria Lopez, Javier
(86) International application number: ES9400122
(87) International publication number: WO9514022

(56) References cited:
- EP-A- 0 406 749
- A. NAPOLITANO ET AL: Oxidation of 4-, 6- A. NAPOLITANO ET AL: Oxidation of 4-, 6- and 7-hydroxyindoles, TETRAHEDRON,vol. 45, and 7-hydroxyindoles, TETRAHEDRON,vol. 45, no. 21, 1989, pages 6749 - 6760 no. 21, 1989, pages 6749 - 6760
- Ullmann's Encyclopedia of Industrial Ullmann's Encyclopedia of Industrial Chemistry, Fifth, Completely Revised Chemistry, Fifth, Completely Revised Edition, Volume A2, pp 50-53 Edition, Volume A2, pp 50-53
- SEYHAN N. EGE, THE UNIVERSITY OF MICHIGAN, SEYHAN N. EGE, THE UNIVERSITY OF MICHIGAN, ORGANIC CHEMISTRY, Structure and ORGANIC CHEMISTRY, Structure and Reactivity, Third Edition, pp. 791-792 Reactivity, Third Edition, pp. 791-792
- P. PERLMUTTER: 'Conjugate Addition P. PERLMUTTER: 'Conjugate Addition Reactions in Organic Synthesis', Reactions in Organic Synthesis', TETRAHEDRON ORGANIC SERIES, PERGAMON PRESS TETRAHEDRON ORGANIC SERIES, PERGAMON PRESS no. 9, 1992, page 2 no. 9, 1992, page 2

## Description

The present invention fits in the technical field of antitumoral agents, that have in their structure a pyrroloindole group.

More particularly, the present invention provides pyrrolo[3,2-e]indole derivatives which have a high antitumoral activity together with a lower toxicity with regard to known compounds.

### PRIOR ART OF THE INVENTION

D.L. Boger et al. (J. Am. Chem. Soc., 113, 2779 (1991) showed that the structural unity of cyclopropa c indol-4-one is responsible for the antitumoral activity of the agent CC-1065 of formula (1):

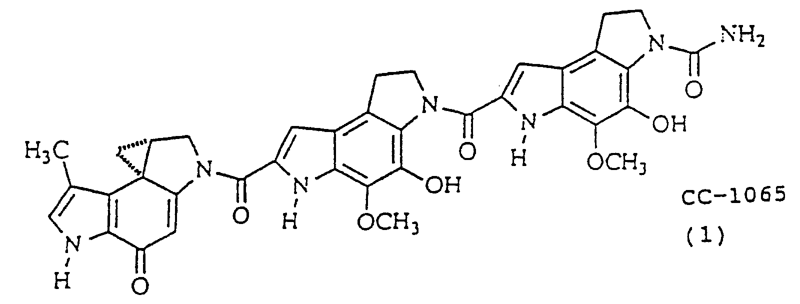

This compound is an extremely strong cytoxine whose biological action is attributed basically to its capacity to covalently bond with structure of the DNA helix ((1) Chidester, C.G.; Krueger, W.C.; Mizsak, S.A.; Duchamp, D.J., Martin, D.G.J. Am. Chem. Soc. 1981, 103, 7629 and references therein. (2) Li, L. H.; Swenson, D. H.; Schpok, S. L.; Kuentzel, S.L.; Dayton, B.D.; Krueger, W. C. Cancer Res. 1982, 42, 999. (3) Swenson, D.H.; Li, L.H.; Hurley, L. H.; Rokem, J. S.; Petzold, G.L.; Dayton, B.D. Wallace, T.L.; Lin, A.H., Krueger, W.C. Cancer Res. 1982, 42, 2821. (4) Bhuyan, B.K.; Newell, K.A.; Crampton, S.L.; von Hoff, D.D. Cancer Res. 1982, 42, 3532. (5) Hurley, L.H.; Reynolds, V.L.; Swenson, D.H.; Petzhold, G.L.; Scahill, T.A. Science (Washington, D.C.) 1984, 226, 843. (6) Reynolds, V.L.; Molineux, L.J., Kaplan, D.J.; Swenson, D.H.; Hurley, L.H. Biochemistry 1985, 24, 6228. (7) Reynolds, V.L.; McGroven, J.P.; Hurley, L.H.J. Antibiot. 1986, 39, 319.)

However, though the compound CC-1065 was, at the beginning, chosen for its development as an anticarcinogenic agent by the NCI ((8) Ducros, J.; Suffness, M.; Cancer Treat. Rev. 1981, 8, 63)), said development was interrupted due to its high toxicity, producing delayed deaths in mice at therapeutic doses ((9) McGovern, J.P.; Clarke, G.L.; Pratt, E.A.; DeKoning, T.F.; J. Antibiot. 1984, 37, 63.)

Therefore, scientific research has been directed towards finding more or less important modifications of the structural formula of CC-1065 that, maintaining its anticarcinogenic capacity, considerably reduced the side effects.

In these lines Adocelesine of formula (2): 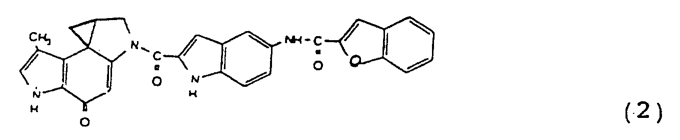 has been synthesized, which not only maintains the high strength of (1) but that also greatly exceeds the natural product in tumoral effectiveness, without provoking delayed lethality ((10) Werpehoski, M.A.. Tetrahedron Letter. 1986, 4103.)

Presently Adolecesine is in phase III of clinical studies ((11) Li, L.H. et al. Investigational New Drugs 9, 137-148, 1991; (12) Smith, K.S. et al., Cancer Chemater. Pharmacol, 30: 348-354, 1992.)

Another compound of interest that, though the studies thereon are not as advanced as those on Adocelesine, but that is very promising is Carcelesine, of formula: 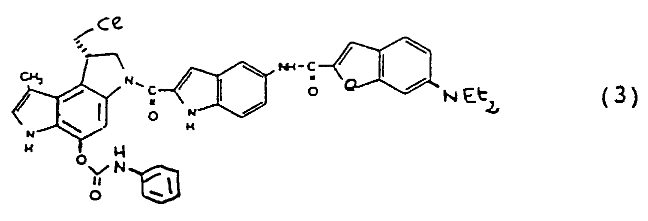 ((13) Li, L.H. et al., Cancer Research, 52, 4904-4913, 1992)

Another compound to be pointed out is CBI-Ind₂, of formula: 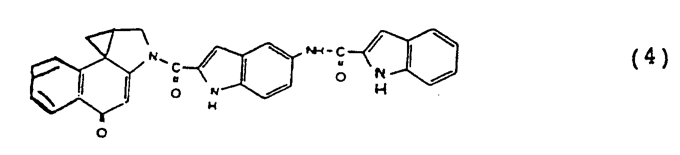 prepared by Boger ((14) Boger, D.L. et al., Bioorganic and Medicinal Chemistry Letters, 1: 115-120, 1991) that shows very good results in the P388 model in vivo.

Likewise, the compounds of formula (5) 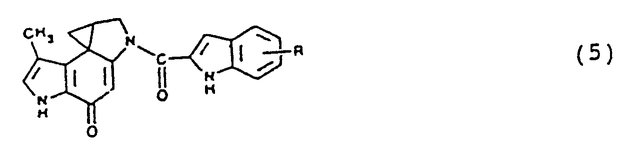 have been obtained, wherein R can have the following meanings:
5- OMe
6-OH, 7-Ome
5-NHCONH²
5-NHCOPh
5-NHCO-2-indolyl
5-NHCO-5-(NHCONH₂)-2-indolyl
54-NHCO-5-(NHCOPh)-2-indolyl
with acceptable anticarcinogenic activity indexes with regard to toxicity ((15) M.A. Warpehoski et al. J. Med. Chem, 1988, Vol. 31, No.3) Further, EP-A-0 406 749 discloses pyrrolo[3,2-e]indole derivatives having anti-tumoral activity and a hydrogen atom at the nitrogen atom of the indole moiety.

However, it is still necessary to advance in the search for compounds structurally related to those cited in the above paragraphs, with a view of introducing other modifications that improve even more the anticarcinogenic/toxicity relationship. In this context, the present invention provides pyrrolo[3,2-e]indole derivatives that optimize said relationship.

### DETAILED DESCRIPTION OF THE INVENTION

The present invention relates to pyrrolo[3,2-e]indole derivatives with anticarcinogenic activity, to processes for the preparation thereof and to its use.

The pyrrolo[3,2-e]indol derivatives of the present invention are characterized in having the following formulae (I), (Ia) and (II): 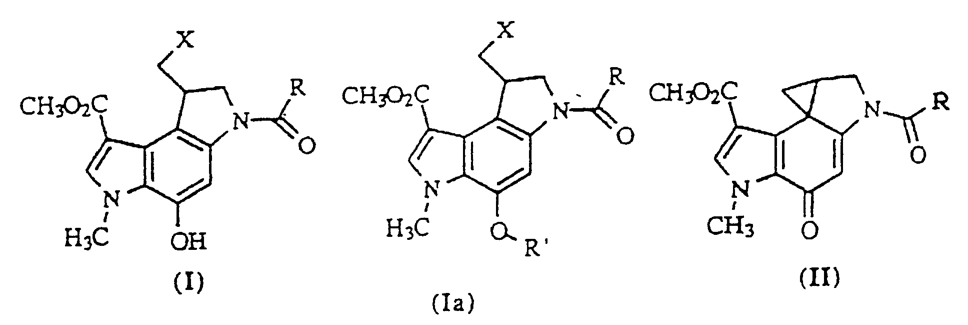 wherein R represents a phenyl, naphthyl, phenanthryl or indolyl group; R' represents an alkanoyl, alkenoyl, alkynoyl, aroyl or heteroaroyl group and X represents chlorine, bromine, iodine.

The preferred meaning for R is indolyl.

The compounds especially preferred of the present invention correspond to the following formula (III) to (VI):

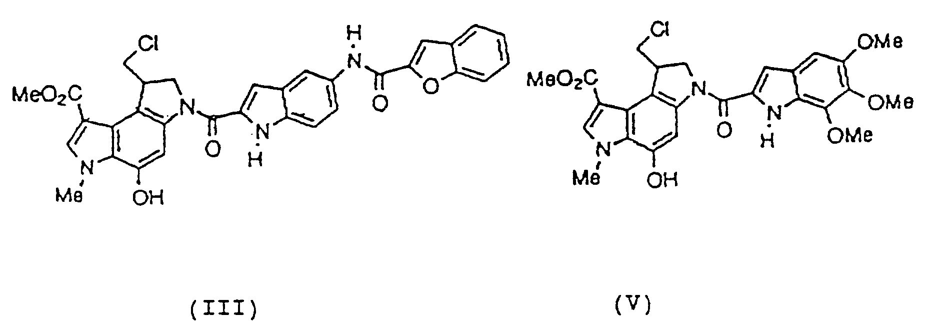

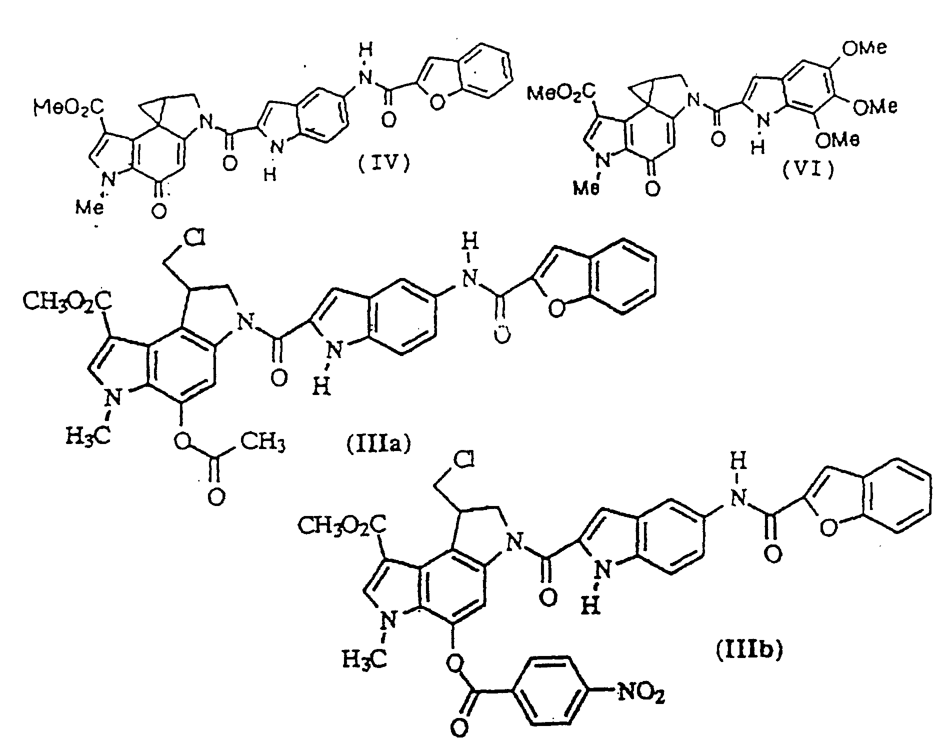

The compounds of formula (I), (Ia) and (II) of the present invention, are obtained from the compound of formula (VII): 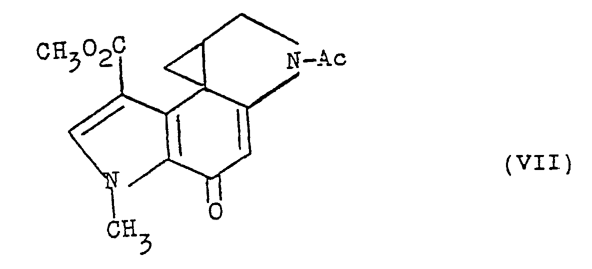 wherein Ac represents an acyl group, generally, acetyl. In a first phase, the compound of formula (VII) is deacylated to produce the compound of formula (VIII):

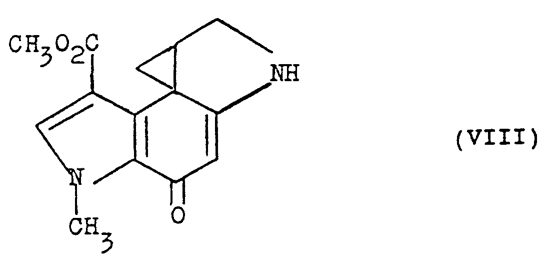

The deacylation reaction is carried out in a basic medium and in a suitable organic solvent. Normally, the base used is an alkoxide, preferably, sodium methoxide and the organic solvent is methanol.

In a second phase, the compound (VIII) is subjected to a reaction to open the cyclopropyl ring to produce a compound of formula (IX): 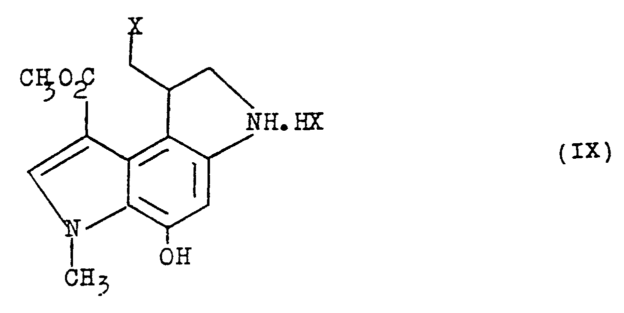 wherein X has the meaning given above for the formula (i.)

The reaction of opening the cyclopropyl ring is carried out by reacting the compound (VIII) with an acid in an organic solvent. The acids to be used, may be hydrogen chloride, hydrogen bromide and hydrogen iodide. As the preferred organic solvent ethyl acetate can be mentioned.

In a third phase, the compound of formula IX) thus obtained is condensed with an acid of formula

R-COOH (X)

wherein R has the meaning given above, or a reactive derivative of the same to produce the active compound of formula (I) indicated above. Condensation is carried out in an organic solvent and in the presence of a condensing agent.

This reaction can be carried out in a aryl carboxylic acid (such as benzoic, naphthoic, phenanthroic acid) or a heteroaryl carboxylic acid (such as indole carboxylic acid) all of them substituted or not substituted.

As an organic solvent to carry out this reaction amides, in particular, N,N-dimethylformamide or N,N-dimethylacetamide, are preferred. As a condensing agent carbodiimides are preferred.

The active compounds of formula (II) indicated above can be obtained from the active compounds of formula (I) in a fourth phase of the processs that comprises treating said compounds of formula (I) with a base in a suitable solvent.

The preferred bases to carry out this transformation are amines and, especially, triethylamine.

As a solvent, a mixture of water with an organic solvent, preferably a mixture of water and acetonitrile, is preferably used.

The active compounds of formula (Ia) can be obtained from the active compounds of formula (I) in a fifth phase of the process that comprises treating said compounds of formula (I) with, alternatively; (a) a carboxylic acid in the presence of a condensing agent; or else, (b) a carboxylic acid chloride in the presence of a base; both alternatives in a suitable solvent.

In alternative (a) an alkanoic, aryl or heteroaryl carboxylic acid substituted or not substituted can be used. As a condensing agent and a solvent those indicated in the transformation of compound (IX) into compound (I) are preferred.

In alternative (b) an alkanoic, aryl or heteroaryl carboxylic acid chloride substituted or not substituted can be used. As a base the use of an amine, preferably triethylamine, is preferred. As a solvent an organic solvent, preferably tetrahydrofuran is used.

The compound of formula (VII), used as a starting product for the process of the present invention, can be obtained in turn from the compound of formula (XI): 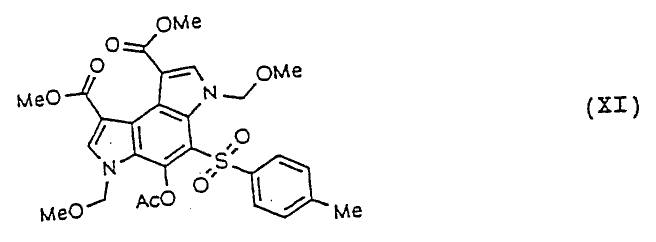 which is one of the products of Spanish patent no. 9201894, by a method already described, but that is summarized in the following paragraphs as a reference.

In a first stage, one of the methoxymethyl groups of compound (XI) is eliminated, by treating it with formic acid at room temperature, to product the compound of formula (XII):

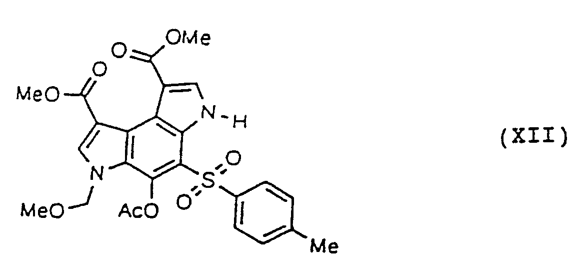

In a second stage, the selective reduction of one of the pyrrolic rings by treating (XII) with Et₃SiH in trifluoroacetic acid is carried out, followed by treatment with acetic anhydride in pyridine to produce a mixture of compounds of formula (XIII) to (XIV):

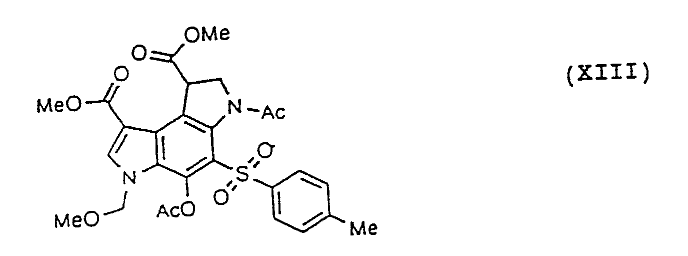

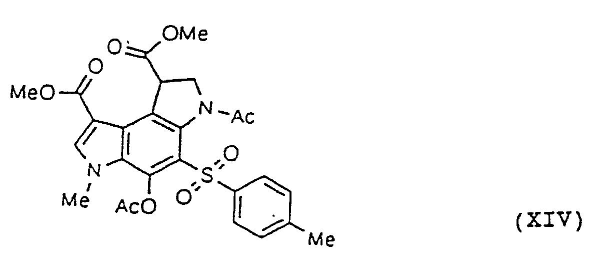

In a third stage, the mixture of (XIII) and (XIV) is subjected to a reaction to eliminate the sulfonyl group by treating the same with sodium and naphthalene in tetrahydrofuran followed by treatment with an acetic anhydride in pyridine, to give a mixture of compounds of formula (XV) and (XVI):

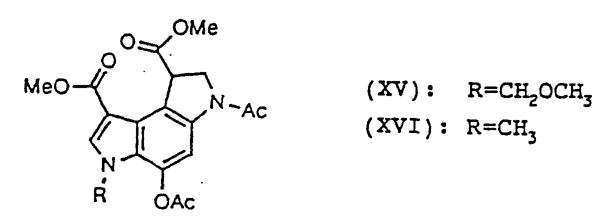

In a fourth stage, the compound of formula (XVI), preferably separated from the mixture, is subjected to a selective reduction of aliphatic ester and O-deacetylation, by reaction with LiAlH₄ in tetrahydrofuran, to produce the compound of formula (XVII):

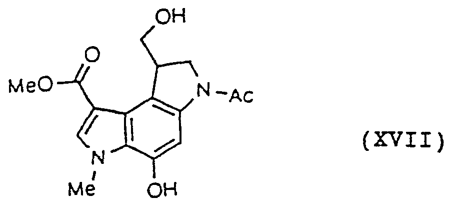

In a fifth stage, the compound (XVII) is subjected to a Mitsunobu-like reaction ((12) O. Mitsunobu, Synthesis 1981, 1) to produce the desired compound of formula (VII).

The compounds of formulae (I) and (II) of the present invention and especially, the compounds of formulae (III), (IV), (V) and (VI) are characterized in that they have a high anti-tumoral activity in vivo together with a very low toxicity, as is manifested in the biological activity studies that will be cited hereinafter. This makes them especially ideal for use as agents for the therapeutic treatment of cancer in its diverse manifestations and, especially, in cases of leukemia.

### EMBODIMENTS OF THE INVENTION

The present invention is additionally illustrated by means of the following Examples.

### PREPARATION EXAMPLE

### Synthesis of methyl 6-acetyl-8-hydroxymethyl-4-ol-3-methyl-3,6,7,8-tetrahydropyrrolo[3,2-e]indole-1-carboxylate

1. Synthesis of dimethyl 4-acetoxy-3-methoxymethyl-5-tosyl-3,6-dihydropyrrolo[3,2-e]indole-1,8-dicarboxylate (XII):

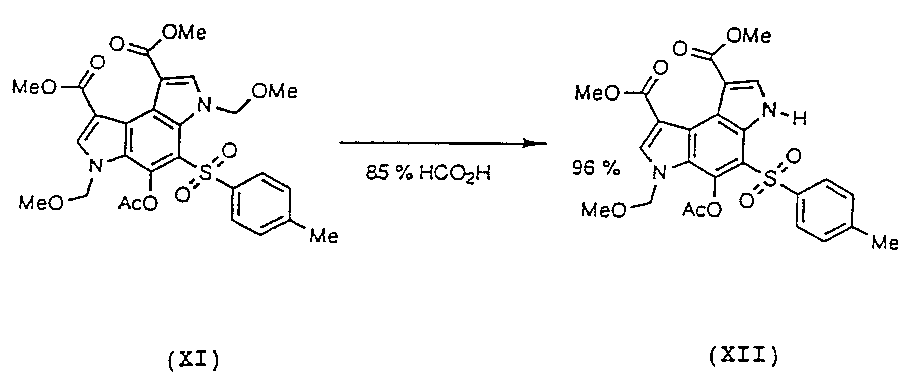

1831 mg of pyrroloindole (XI) (3.20 mmol) were dissolved in 30 mL formic acid 85%, maintaining agitation at room temperature for 24 hours.

To elaborate, water (250 mL) was added, extraction was carried out with CH₂Cl₂ (3x75 mL) and the organic phase was dried with anhydrous NaSO₄. Finally, the residue obtained upon evaporating the solvent was purified by chromatography in a silica flash gel column (16x2 cm ⌀, hexane:EtOAc gradient from 50 to 60% EtOAc), allowing to obtain after vacuum drying, 1623 mg (96%) of the mono-unprotected compound (XII.)
m.p.: 135-137ºC (EtOAc:hexane). Rf.: 0.52 (CH₂Cl₂:EtOAc 17:3)
IR (NaCl, νₘₐₓ): 1725, 1790, 2950, 3410 cm⁻¹
UV (Ethanol, λₘₐₓ): 206, 242, 268, 328 nm
¹H-NMR(CDCl₃): 2.33 (s, 3H, ArOCOCH₃), 2.42 (s, 3H, ArCH₃), 3.18 (s, ArCH₂OCH₃), 3.81 (s, 3H, ArCO₂CH₃), 3.82 (s, 3H, ArCO₂CH₃), 5.02 (d, 1H, J=10.8 Hz, ArCH₂OCH₃). 5.87 (d, 1H, J=10.8 Hz, ArCH₂OCH₃), 7.21 (d, 2H, J=8.3 Hz, ArH), 7.78 (d, 2H, J=8.2 Hz, ArH), 7.79 (s, 1H, ArH), 7.92 (d, 1H, J=2.9 Hz, ArH), 10.68 (s wide, 1H, ArNH).
¹³C-NMR(CDCl₃): 21.4, 21.45, 51.3, 51.4, 55.3, 80.3, 111.2, 111.5, 113.0, 116.8, 125.3, 125.8, 126.5, 129.1, 129.8, 130.6, 134.5, 137.4, 139.7, 144.7, 165.4, 165.8, 169.2
Mass spectrum (m/e, %): 528 (M^{+.},4), 497 (M^{+.}-CH₃O,5), 486 (M^{+.}CH₂CO, 100), 454 (M^{+.}CH₂CO-CH₃OH, 58). 33^{º} (M^{+.} -CH₂CO-CH₃(C₆H₄)SO₂.7), 299 (M^{+.}-CH₂CO-CH₃(C₆H₄)SO₂-CH₃OH. 3), 139 (CH₃(C₆H₄)SO^{-.}, 13) 91 (CH₃(C₆H₄)^{+.},12).
Mass spectrum (high resolution) for C₂₅H₂₄N₂O₉S: Calculated: 528.1202: Found: 528.1215.
Elementary analysis for C₂₅H₂₄N₂O₉S:
Calculated: % C=56.81; % H=4.58; % N=5.30, % S= 6.07
Found: % C=56.77; H=4.64; % N=5.23; % S=6.09

2. Reduction of dimethyl 4-acetoxy-3-methoxymethyl-5-tosyl-3,6-dihydropyrrolo[3,2-e]indole-1,8-dicarboxylate (XII) with Et₃SiH in an acid medium.

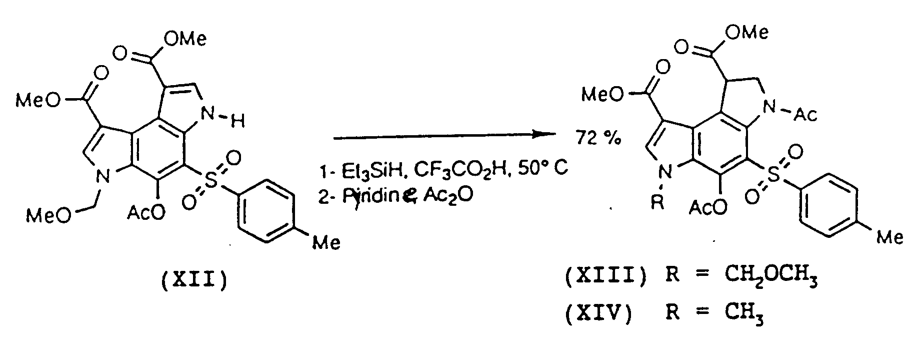

4.5 ml of trifluoroacetic acid (58.41 mmol) was added to a mixture of the pyrroloindole (XII) (500 mg. 0.946 mmol) and Et₃SiH (0.9 mL, 5.65 mmol) under argon, heating the resulting mixture in a paraffin bath at 60ºC for 8 hours. Then, the mixture was cooled in a bath at -50º C and 9 mL of pyridine (115 mmol), 3 ml of acetic anhydride (31.73 mmol) and 3 mL of CH₂Cl₂ were added, heating again at 60º C for 4.5 hours.

Adding HCl 10% (75 mL) to the reaction mixture, followed by extraction with CH₂Cl₂ (3x20 mL), the organic phase washed with a saturated Cu-SO₄ solution, dried with anhydrous NaSO₄ and concentrated at reduced pressure, led to a solid residue, that was purified by chromatography in a silica flash gel column (20x2 cm ⌀, CH₂Cl₂ to CH₂Cl₂:ETOAc gradiet 17:3, yielding after vacuum drying 389 mg of a non-separated mixture of products (XIII) and (XIV).

Spectoscopic data of the mixture:
RF.: 0.36 (CH₂Cl₂:EtOAc 7:3).
IR(NaCl,νₘₐₓ): 1680, 1720, 1740, 1790, 2960 cm⁻¹
UV (Ethanol, λₘₐₓ): 204, 224, 266, 338 nm
Mass spectrum (m/e, %): 572 (M^{+.}, 0.2), 530 (M^{+.}, -CH₂CO, 3), 514 (M^{+.}-CH₂OCO, 2), 472 (M^{+.}-CH₂CO-CH₂OCO, 32), 430 (M^{+.}-CH₂CO-CH₂OCO-CHCO-CH₂CO-CH₂OCO, 100), 317 (M^{+.}-CH₂CO-CH₂-OCO-CH₃(C₆H₄)SO₂, 60), 284 (M^{+.}-CH₂CO-CH₃ OCO-CH₃(C₆H₄)SO₂-CH₃OH, 63), 242 (M^{+.}-CH₂COCH₃OCO-CH₃(C₆ H₄)SO₂-CH₂CO-CH₃OH. 58). 139 (CH₃(C₆H₄)SO_{+.}, 38) 91 (CH₃(C₆H₄)^{+.}, 50).
Mass spectrum (FAB) (m/e, %): 573 (M+1,3), 531 (M+1-CH₂CO, 23), 515 (M+1-CH₂OCO, 24), 501 (M*+1-CH₂CO, 7) 488 (M+1-CH₂CO-CH₃CO, 23), 472 (M+1-CH₂CO-CH₃OCO, 24), 430(M+1-CH₂ CO-CH₂CO, 100), 400 (M*+1-CH₂CO-CH₃OCO-CH₂CO, 26), 418 (M+1-CH₃(C₆H₄)SO_{2.} 10), 399 (M+1-CH₂CO-CH₃OCO-CH₂CO-CH₃O, 44), 367 (M+1-CH₂CO-CH₃OCO-CH₂CO-CH₃O-CH₃OH, 25), 360 (M+1-CH₂OCO-CH₃(C₆H₄)SO₂, 35), 318 (M+1-CH₂OCO-CCH₃(C₆ H₄)SO₂-CH₂CO, 24).

3. Treating the mixture of pyrroloindoles (XIII) and (XIV) with sodium and naphthalene. 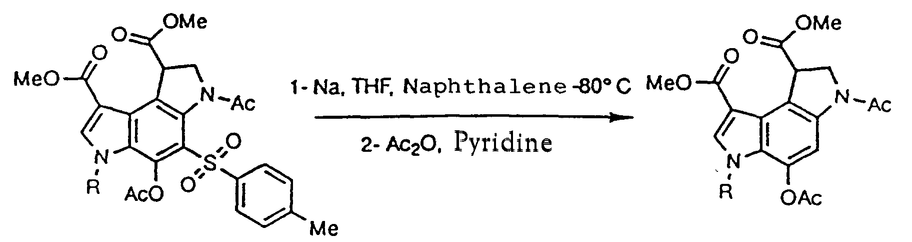 (XIII) R = CH₂OCH₃ (XV) R = CH₂OCH₃ Traces (XIV) R = CH₃ (XVI) R = CH₃ 28 % from (XII)

A mixture of naphthalene (320 mg. 2,49 mmol) and sodium (55 mg, 2.39 mmol) in dry THF (10 mL), was maintained with agitation at -15º C under argon for 2.5 hours. Afterwards the reaction mixture was cooled to -80º C and the mixture of pyrroloindoles (XIII) and (XIV) (105 mg. 0.194 mmol) was added to it, maintaining agitation until the starting substances disappear by TLC (10 minutes), moment in which the acetic anhydride (1 mL, 10.57 mmol) and pyridine (1 mL, 12.39 mmol) are added, passing in turn the reactoin mixture at room temperature and leaving it with agitation and under argon for 24 hours.

Acidifying with HCl 10% (20 mL) of the reaction mixture, followed by extraction with EtOAc (3x10 mL), the organic phase washed with a saturated CuSO₄ solution, dried with anhydrous Na₂SO₄ and purified by chromatography in a silica flash gel column (14x2 cm ⌀, CH₂Cl₂:EtOAc gradient from 30 to 40% in EtoAc), yielded 35 mg of a mixture of homogenous products by TLC. This mixture was purified by HPLC (isopropanol:hexanol gradient from 40 to 20% in hexane, flow 3.5 mL/min and detection at 275 nm), separating after vacuum drying, 28 mg. of pyrroloindole (XVI) (28% in two stages, from the compound (XII), along with traces of pyrroloindole (XV.)

Spectroscopic data of pyrroloindole (XVI):
m.p.: 223-224º C (methanol), Rf.: 0.36 (CH₂Cl₂:EtOAc 7:3:)
IR (NaCl,νₘₐₓ) 1655, 1705, 1735, 1765, 2960, 3120 cm⁻¹
UV (Ethanol, λₘₐₓ): 214_{hb}, 254, 298 nm
¹H-NMR(CDCl₃): 2.20 (s, 3H, RCOCH₃), 2.35 (s, 3H, RCOCH₃), 2.35 (s, 3H, RCOCH₃), 3.63 (s, 3H, ArCH₃), 3.76 (s, 3H, ArCH₃), 3.76 (s, 3H, ArCO₂CH₃, 3.84 (s, 3H, ArCo₂CH₃), 4.31 (m, 2H, RCH₂CH(R)₂), 5.02 (dd. 1H, J=5.1 and 9.5 Hz, RCH₂CH_{R}2), 7.62 (s, 1H, ArH), 8.08 (s, 1H, ArH).
¹³C-NMR(CDCl₃): 20.8, 24.0, 36.1, 46.0, 50.9, 52.1, 52.8, 106.7, 107.6, 117.0, 125.7, 126.6, 136.7, 138.4, 139.1, 164.5, 167.9, 169.4, 173.0.
Mass spectgrum (m/e, %): 388 (M^{+.}, 10), 346 (M^{+.}-CH₂CO, 9) 330 (M^{+.}-CO₂CH₂, 25), 213 (M^{+.}-CO₂CH₂-CH₂CO-CH₃CO-CH₃OH, 51), 197 (M^{+.}-CO₂CH₂-CH₂CO-CH₃CO-CH₃OH-CH₃-H, 100).
Mass spectrum (high resolution) for C₁₉H₂₀N₂O₇: Calculated: 388.1270; Found: 388.1274.

4. Treating the mixture of pyrroloindoles (XIII) and (XIV) with sodium and N,N-dimethyl-1-naphthylamine. 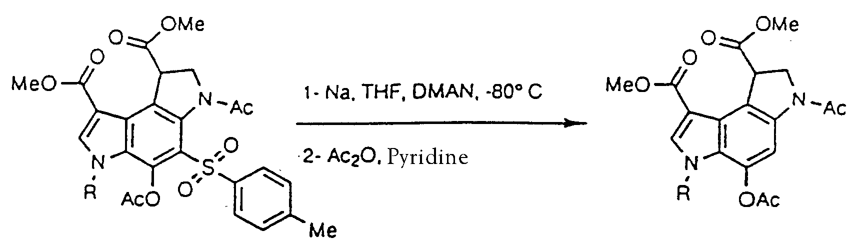 (XIII) R = CH₂OCH₃ (XV) R = CH₂OCH₃ Traces (XIV) R = CH₃ (XVI) R = CH₃ 26 % from (XII)

A mixture of N,N-dimethyl-1-naphthylamine (0.3 mL, 1,827 mmol) and sodium (25 mg. 1.087 mmol) in dry THF (5 mL) was kept with agitation under argon in a bath at -15º C for 1.5 hours. Then the reaction mixture was cooled at -80ºC and the mixture of the compounds (XIII) and (XIV) was added to it, maintaining agitation in the bath for 20 minutes. To the reaction mixture at this temperature 0.8 ml of acetic anhydride (8.46 mmol) and 0.5 mL of pyridine (6.19 mmol) were then added, passing the mixture at room temperature and maintaining the reaction for 40 hours.

Adding HCl 10 % (10 mL) to the reaction mixture, followed by extraction with EtOAc (3x4 mL), the organic phase washed with a saturated CuSO₄ solution, dried with anhydrous Na₂SO₄ and purified by chromatography in a silica flash gel column (15x1.5 cm ⌀, EtOAc:hexane gradient from 10 to 0% in hexane), provided 33 mg of a mixture which after purification HPLC (isopropanol:hexane gradient from 40 to 20/ in hexane, flow 3.5 mL/min and detection at 275 nm) yielded after vaccum drying 26 mg of the pyrroloindole (XVI) (26% in two stages, from compound (XII), along with traces of the pyrroloindole (XV),

5. Synthesis of methyl 6-acetyl-8-hydroxymethyl-4-ol-3-methyl-3,6,7,8-tetrahydropyrrolo[3,2-e]indole-1-carboxylate

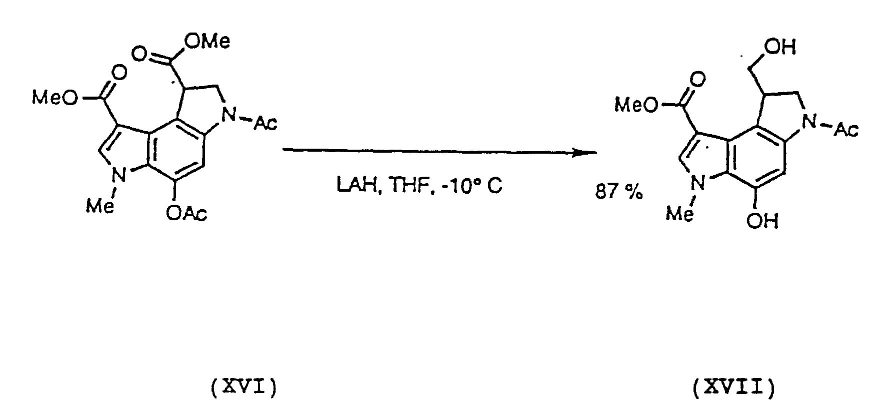

0.32 mL of a solution of LiAlH₄ in THF (1 M, 0.32 mmol) was added to a solution agitated under argon and cooled to -10º C of pyrroloindole (XVII) (42 mg. 0.108 mmol) in dry THF, maintaining the agitation at a low temperature for 30 minutes.

Adding EtOAc (2 ml) to the reaction mixture, followed by acidifying with HCl 10 %, adding a saturated NaCl solution (5 ml), extracting with EtOAc (5x4 mL), drying with anhydrous Na₂SO₄ and eliminating the solvent, yielded a solid residue that was passed through a slica flash gel column (18x1 cm ⌀), eluting with EtOAc:methanol (9:1), yielding 30 mg (87%) of the diol (XVII).
m.p.: 201-205º C (dry) (EtOAc). Rf.: 0.20 (EtOAc).
IR (KNr, νₘₐₓ): 1610, 1636m 1674m 1702, 3121, 3434 cm⁻¹
UV (Ethanol, λₘₐₓ): 254, 312 nm.)
¹H-NMR(DMSO-D₆): 2.15 (s, 3 H, ArCOCH₃), 3.00 (m, 1H, R₂CHR), 3.67 (m, 1H, RCH₂NR₂), 3.73 (s, 3H, ArCH₃), 4.00 (s+m, 5H, ArCO₂CH₃ and RCH₂OH), 4.12 (m, 1H, RCH₂ NR₂), 4.70 (s wide, 1 H, RCH₂OH), 7.75 (s, 1H, ArH), 7.91 (s, 1H, ArH), 10.00 (s wide, 1H, ArOH).
Mass spectrum (m/e, %): 318 (M^{+.}, 31), 300 (M^{+.}-H₂O, 5), 287 (M^{+.}-HOCH₂, 74), 255 (M^{+.}-HOCH₂-CH₂-CH₃OH, 21), 228 ((M^{+.}-HOCH₂-CO₂CH₃, 8) 213 (M^{+.}-HOCH₂-CH₂CO-CH₃OH, 100) 186 (M^{+.}-HOCH₂CO₂CH₃-CH₂CO, 19).
Mass spectrum (high resolution for C₁₆H₁₈N₂O₃: Calculated: 318.1216; Found: 318.1223

6. Synthesis of methyl 2-acetyl-5-methyl-4-oxo-1,2,4,5, 8,8a-hexahydrocyclopropa[c]pyrrolo[3,2-e]indole-7-carboxylate (VII)

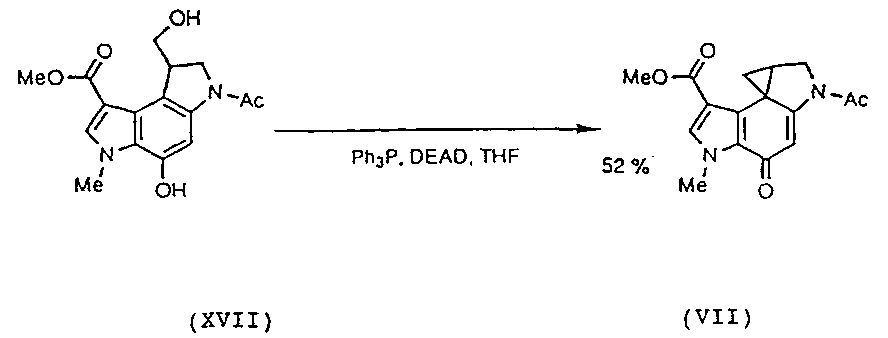

0.03 mL of diethyl azodicarboxylate (0.189 mmol) were added to a magnetically agitated solution under argon of the diol (XVII) (27 mg. 0.085 mmol) and triphenylphosphine (74 mg. 0.282 mmol) in dry THF, maintaining agitation for 19 hours.

After purifying by chromatography in a silica flash gel column (18x1 cm ⌀) eluting with EtOAc:hexane (17:3, of the residue obtained by eliminating the solvent at reduced pressure, 105 mg of a triphenylphosphine oxide and reaction product mixture were isolated. Finally, by purifying this mixture by HPLC (isopropanol:hexane gradient from 35 to 20 % in hexane, flow 3.5 mL/min and detection at 254 nm) 13 mg (52%) of the analogue (VII) of the fragment (CPI) were isolated.
m.p.: 193-195º C (CH₂Cl₂:hexane). RF.: 0.41 (EtOAc).
IR(KBr): 1595, 1616, 1688, 2948 cm⁻¹
UV (Ethanol, λₘₐₓ): 220, 244, 280, 294, 330 nm
¹H-NMR(CD₂Cl₂): 1.19 (m, 1H, RCH₂R), 2.17 (s+m, 4H, ArCOCH₃ and RCH₂R), 3.49 (m, 1H, R₂CHR), 3.72 (s, 3H, ArCH₃), 4.00 (s+m, 5H, ArCO₂CH₃ and RCH₂NR₂), 6.83 (s, wide, 1H, RCOCHR), 7.36 (s, 1H, ArH).
¹³C-NMR(CDCl₃), 23.9, 24.3, 24.5, 32.2, 37.1, 51.1, 53.2, 108.9, 111.5, 129.6, 132.0, 134.2, 159.5, 163.8, 170.0, 178.5.
Mass spectrum (m/e, %): 300 (M^{+.}, 26), 269 (M^{+.}-CH₃O.3), 257 (M^{+.}-CH₃CO, 40), 243 (M^{+.}-CH₃CO-CH₂, 22), 225 (M^{+.}-CH₃CO-CH₃OH, 19), 198 (M^{+.}-CH₃CO-CH₃OCO, 19), 43(CH₃CO^{+.}, 100).
Mass spectrum (high resolution) for C₁₆H₁₆N₂O₄: Calculated: 300.1110; Found: 300.1097.

### EXAMPLES OF SYNTHESIS

### EXAMPLE 1

### Synthesis of methyl 8,chloromethyl-4-hydroxy-3-methyl-3, 6,7,8-tetrahydropyrrolo[3,2-e]indole-1-carboxylate hydrochloride (IX)

### (a) Synthesis of methyl 5-methyl-4-oxo-1,2,4,5,8,8a-hexahydrocyclopropa[c]pyrrolo[3,2-e]indole-7-carboxylate (VII).

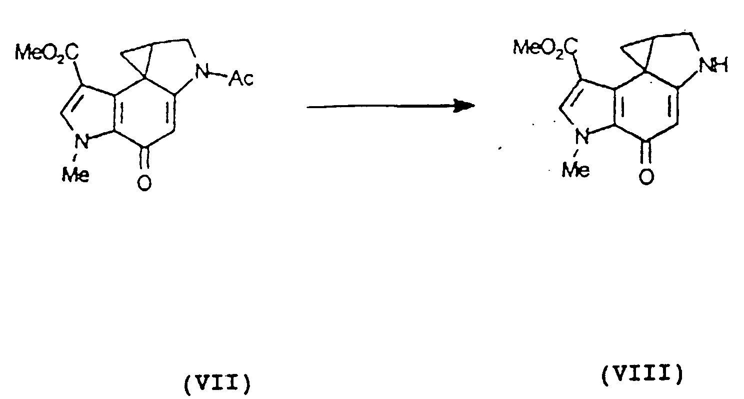

0.2 mL of a 1.25 M sodium methoxide solution in methanol were added to a solution agitated magnetically under argon of the compound (VII) (13 mg. o.043 mmol) in dry methanol (4 mL), maintaining the agitation at room temperature for 10 minutes.

After purifying in a silica flash gel column (10x1 cm ⌀). the residue obtained by eliminating the solution under reduced pressure, eluting with EtOAC, 11 mg (99%) of the compound (VIII) were isolated.
RF.: 0.26 (EtOAc).
¹H-NMR(CD₂Cl₂): 1.11 (dd, 1H, J=2.9 and 4.8 Hz, RCH₂R), 2.04 (dd, 1H, J=2.8 and 7.8 Hz, RCH₂R), 3.48 (m, 1H, R₂CHR), 3.54 (d 1H, J=10.2 Hz, RCH₂R), 3.71 (m+s, 4H, RCH₂R and ArCH₃), 3.98 (s, 3H, ArCO₂CH₃), 5.18 (s wide, 1H, NH), 5.37 (s, 1H, RCOCHR), 7.25 (s, 1H, ArH).
Mass spectrum (m/e, %): 258 (M_{+.}, 100), 243 (M^{+.}-CH₃, 17), 225 (M^{+.}-CH₃OH-H, 32), 199 (M^{+.}-CO₂CH₃, 40).

### (b) Synthesis of methyl 8-chloromethyl-4-hydroxy-3-methyl-3,6,7,8-tetrahydropyrrolo[3,2-e]indole-1-carboxylate hydrochloride (IX).

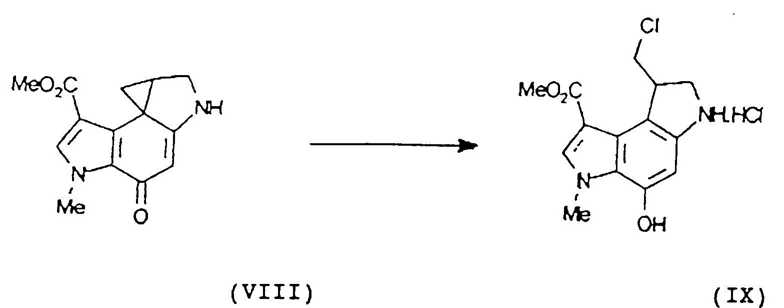

For 30 minutes, the anhydrous hydrogen chloride was bubbled through a solution of compound (VIII) (11 mg., 0.042 mmol) in dry ethyl acetate (3mL), keeping the reaction at room temperature.

The resulting yellow suspension was concentrated at reduced pressure and vacuum dried, permitting the isolation of 13 mg (93%) of compound (IX.)

### EXAMPLE 2

### Synthesis of methyl 6-{5-[(benzofuran-2-ylcarbonyl)-amino]1H-indole-2-carbonyl}-8-chloromethyl-4-hydroxy-3-methyl-3,6,7,8,tetrahydropyrrolo[3,2-e]indole-1-carboxylate (III)

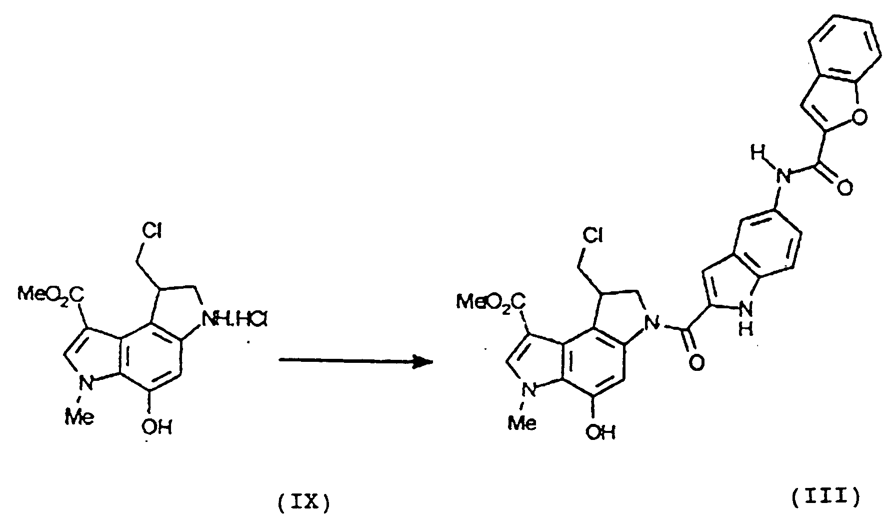

A mixture of the compound (IX) (21 mg., 0.063 mmol), of 5- (benzofuran-2-ylcarbonyl)amino -lH-indol-2-carboxylic acid (84 mg, 0262 mmol) and N-(3,3-dimethylaminopropyl)-N-ethylcarbodiimde hydrochloride (79 mg, 0.412 mmol) in dry DMF (2mL), was maintained with agitation at room temperature and under argon for 24 hours.

Adding water (5 mL) and brine (2 mL) to the reaction mixture, followed by extracting with EtOAc (4x5 mL), the organic phase dried with anhydrous Na₂SO₄ and purifying by chromatography in a silica flash gel column (14x1.5 cm ⌀) eluting with EtOAc:Hexane (17:3), made it possible to obtain after vacuum drying, 26 mg. (69%) of compound (III).
Rf.: 0.60 (Hexane:acetone 1:1)
1H:NMR (Acetone-D₆:DMSO-D₆ 9:1): 3.49 (dd, 1H, J = 8.9 and 10.3 Hz, R₂CHCH₂NR₂), 3.81 (s, 3H, ArCH₃), 3.99 (dd, 1H, J=3.1 and 10.3 Hz, R₂CHCH₂NR₂), 4.12 (s, 3H, ArCO₂CH₃), 4.48 (m, 1H, R₂CHCH2Cl), 4.67 (m, 2 H, R₂CHCH₂Cl), 7.19 (d, 1H, J=1.6 Hz, ArH), 7.35 (dt, 1H, J=1.0 and 7.45 Hz, ArH), 7.51 (m, 2H, ArH), 7.68 (m, 3H, ArH), 7.81 (dd, 1H, J=1.0 and 7.5 Hz, ArH), 7.92 (s, 1H, ArH), 7.95 (s, 1H, ArH), 8.34 (d, 1H, J=1.7 Hz, ArH), 10.06 (s, 1H, ArOH), 10.21 (s, 1H, ArNHCOAr), 10.24 (s, wide, 1H, ArNH).

### EXAMPLE 3

### Synthesis of methyl 2-[5-[(benzofuran-2-ylcarbonyl) amino]-1H-indol-2-carbonyl]-5-methyl-4-oxo,1,2,4,5,8,8,a-hexahydrocyclopropa[c]pyrrolo[3,2-e]indole-7-carboxylate (IV).

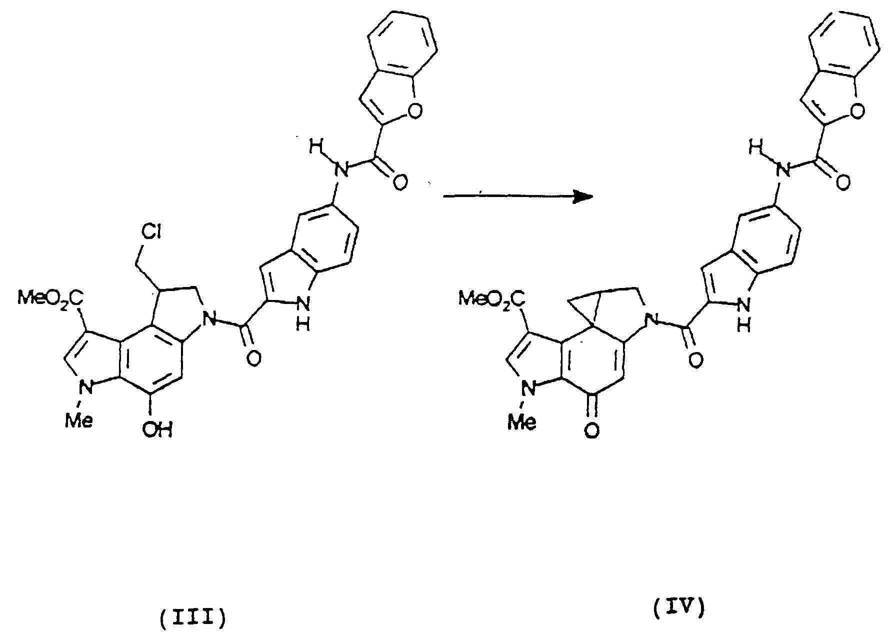

Compound (III) was dissolved in 3.5 mL of a mixture of acetonitrile:water:triethylamine (5:1:1), maintaining agitation at room temperature for 1 hour.

The resulting solution was diluted in EtOAc (50 mL), washed with water (3x15 mL), dried with anhydrous Na₂SO₄ and purified by chromatography in a silica flash gel column (9x1, cm ⌀), eluting with acetone:hexane (1:1). After vacuum drying 8 mg. (95%) of compound (IV) were obtained.
Rf.: 0.55 (Acetone:hexane 3:2).
1H:NMR (CD₂Cl₂:DMSO-D₆ 9:1): 1.26 (m 1H, RCH₂CHR₂), 2.14 (dd, 1H, J=3.2 and 7.5 Hz, RCH₂CHR₂), 3.57 (m, 1H, RCH₂CHR₂) 3.65 (s, 3H, ArCH₃), 3.93 (s, 3H, ArCO₂CH₃), 4.35 (m, 2H, RCH₂NR₂), 6.77 (s, 1H, ArH), 6.93 (s, 1H, ArH), 7.22 (t, 1H, J=7.6 Hz, ArH), 7.37 (m, 2H, ArH), 7.49 (m, 2H, ArH), 7.63 (d, 1H, J=7.8 Hz, ArH), 8.11 (s, 1H, ArH), 9.66 (s, 1H, ArNHCOAr), 11.35 (s, 1H, ArNH).

### EXAMPLE 4

### Synthesis of methyl 6-(5,6,7-trimethoxy-1H-indole-2-carbonyl)-8-chloromethyl-4-hydroxy-3-methyl-3,6,7,8-tetrahydropyrrololo[3,2-e]indole-1-carboxylate (V).

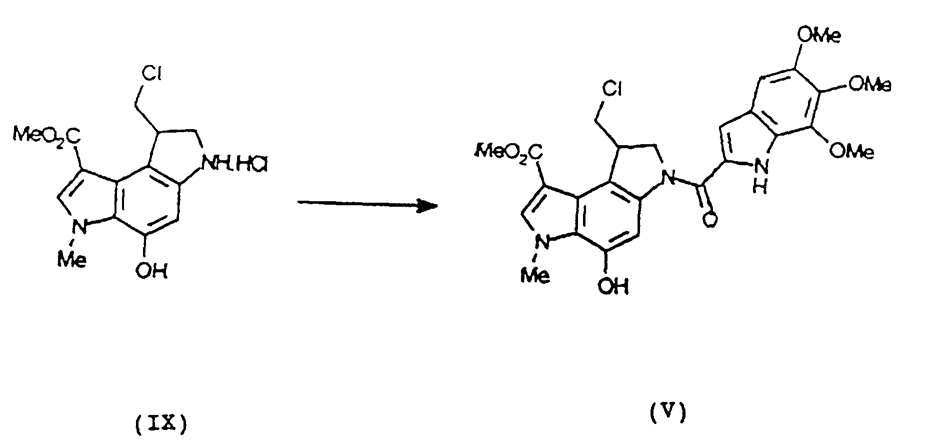

A mixture of compound (IX) (13 mg, 0.039 mmol), 5,6,7-trimethoxy-1H-indole-2-carboxylic acid (35 mg., 0.144 mmol) and N-(3,3-dimethylaminopropyl)-N-ethylcarbodiimide hydrochloride (46 mg, 0.24 mmol) in dry DMF (1.5 mL), was maintained with agitation at room temperature and under argon for 24 hours.

Adding water (5 mL) and brine (2 mL) to the reaction mixture, followed by extracting with EtOAc (4x5 mL), the organic phase dried with anhydrous Na₂SO₄ and purifying by chromatography in a silica flash gel column (13x1 cm ⌀) eluting with EtOAc:hexane (17:3), made it possible to obtain after vacuum drying, 18 mg (87%) of compound (V).
Rf.: 0.65 (Hexane:acetone 1:1)
¹H-NMR (Acetone-D₆): 3.46 (m, 1H, R₂CHCH₂NR₂, 3.80 (s, 3H, RCH₃), 3.85 (s, 3H, RCH₃) 3.86 (s, 3H, RCH₃), 3.98-4.00 (m, 1H, R2CHCH₂NR₂), 4.01 (s, 3H, RCH₃), 4.13 (s, 3H, RCH₃), 4.58 (m, 3H, R₂CHCH₂Cl and R₂CHCH₂Cl), 6.96 (s, 1H, ArH), 7.08 (d, 1H, J=2.2 Hz, ArH), 7,86 (s, 1H, ArH), 7.94 (s, 1H, ArH), 9.22 (s wide, 1H, ArNH), 10.28 (s, wide, 1H, ArOH).

### EXAMPLE 5

### Synthesis of methyl 2-(5,6,7-trimethoxy-1H-indole-2-carbonyl)-5-methyl-4-oxo-1,2,4,5,8a-hexahydrocyclopropa[c]pyrrolo[3,2-e]indole-7-carboxylate (VI)

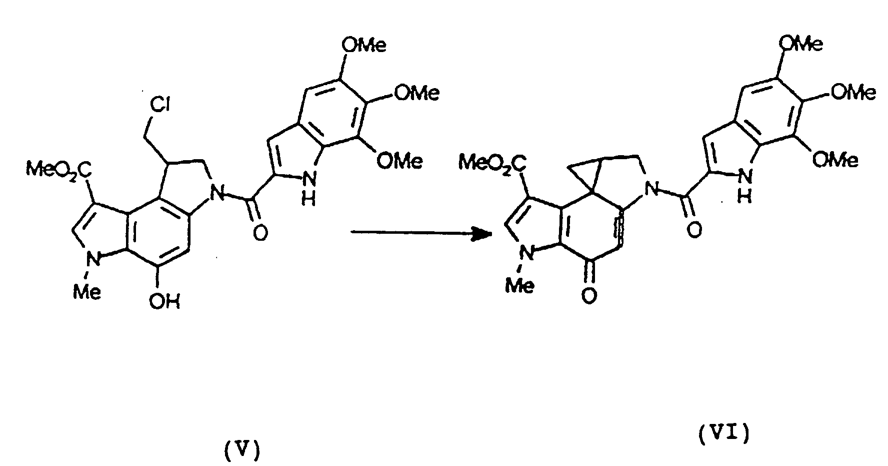

Compound (V) was dissolved in 2.5 mL of a mixture of acetonitrile:water:triethylamine (3:1:1), maintaining the agitation at room temperature for 1 hour.

The resulting solution was diluted with EtOAc (50 mL), washed with water (3x15 mL), dried with anhydrous Na₂SO₄ and purified by chromatography in a silica flash gel column (12x1 cm ⌀), eluting with EtOAc:hexane (4:1). After vacuum drying 13 mg. (83%) of compound (VI) were obtained.
Rf.: 0.45 (Acetone:hexane 1:1).
¹H-NMR(CD₂Cl₂): 1.32 (1H, RCH₂CHR₂). 2.24 (dd, 1H, J=3.5 and 7.6 Hz, RCH₂CHR₂), 3.61 (m, 1H, RCH₂CHR₂). 3.75 (s, 3H, RCH₃), 4.02 (s, 3H, RCH₃), 4.03 (s, 3H, RCH₃). 4.034 (s, 3H, RCH₃), 4.40 (d, 2H, J=2.6 Hz, RCH₂NR₂), 6.82 (s, 1H, ArH), 6.91 (s, 1H, ArH), 6.93 (d, 1H, J=2.3 Hz, ArH), 7.39 (s, 1H, ArH), 9.34 (s wide, 1H, ArNH).

### EXAMPLE 6

### Synthesis of methyl 4-acetoxy-6-{5-[(benzofuran-2-yl-carbonyl)-amino]1H-indole-2-carbonyl}-8-chloromethyl-3-methyl-3,6,7,8-tetrahydropyrrolo[3,2-e]indole-1-carboxylate (IIIa)

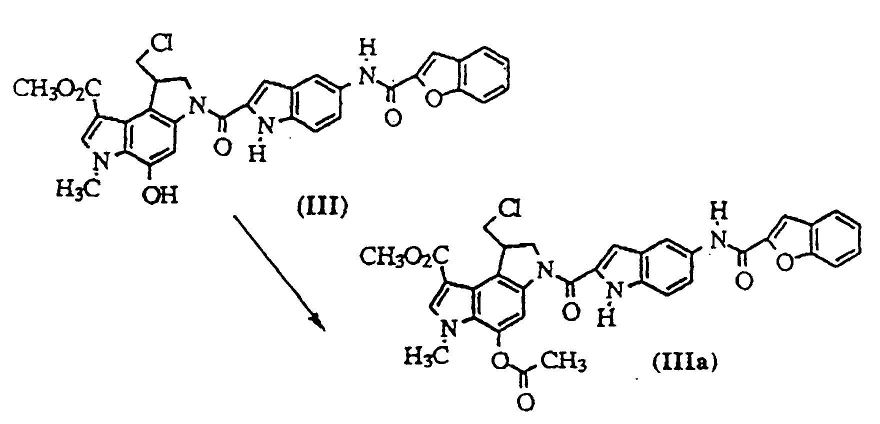

A solution of acetyl chloride (0,024 mL, 0.034 mmol) in dry tetrahydrofuran (2 mL) was added to a solution, cooled to -20º C and agitated under argon of compound (I) (10 mg, 0.017 mmol) and triethylamine (0.047 mL, 0.34 m mmol) in dry tetrahydrofuran (3 mL).

Half an hour later the resulting solution was passed at room temperature and brine (2 mL) and hydrchloric acid (10%) (2mL) were added. The solution resulting from extraction with ethyl acetate (3X8 mL) was dried (Na₂SO₄) and concentrated, yielding a residue that was purified by chromatography in a silica flash gel (12X1.5 cm ⌀), eluting with CH₂Cl₂:EtOAc (4:1.) 9 mg. (83% of compound (IIIa) were obtained.
m.p.: 189-191ºC (EtOAc). Rf.: 0.60 (hexane:acetone, 1:1).
¹H-NMR(DMSO-D₆): 2.42 (s, 3H, CH₃CO). 3.65 (dd, 1H, J=8.2 and 10.7 Hz, R₂CHCH₂NR₂), 3.80 (s, 3H, ArCH₃), 3.92 (s, 3H, ArCO₂CH₃), 3.97 (m, 1H, R₂CHCH₂NR₂), 4.50 (m, 1H, R₂CHCH₂Cl), 4.62 (d, 1H, J= 10.7 Hz, R₂CHCH₂Cl), 4.76 (m, 1H, R₂CHCH₂Cl), 7.19 (s, 1H, ArH), 7.36 (t, 1H, J= 7.8 Hz, ArH), 7.48 (d, 1H, J= 8.8 Hz, ArH), 7.50 (m, 1H, ArH), 7.60 (dd, 1H, J=1.0 and 8.0 Hz, ArH), 7.72 (d, 1H, J=8.1 Hz, ArH), 7.75 (s, 1H, ArH), 7.82 (d, 1H, J= 7.8 Hz, ArH), 8.02 (s, 1H, ArH), 8.18 (s, 1H, ArH), 8.19 (s, 1H, ArH), 10.45 (s, 1H, ArNHCOAr), 11.67 (s, 1H, ArNH).

### EXAMPLE 7

### Synthesis of methyl 6-{5-[(benzofuran-2-ylcarbonyl)amino 1H-indole-2-carbonyl}-8-chloromethyl-3-methyl-4-(4-nitrobenzoyl)-3,6,7,8-tetrahydropyrrolo[3,2-e]indole-1-carboxylate (IIIb)

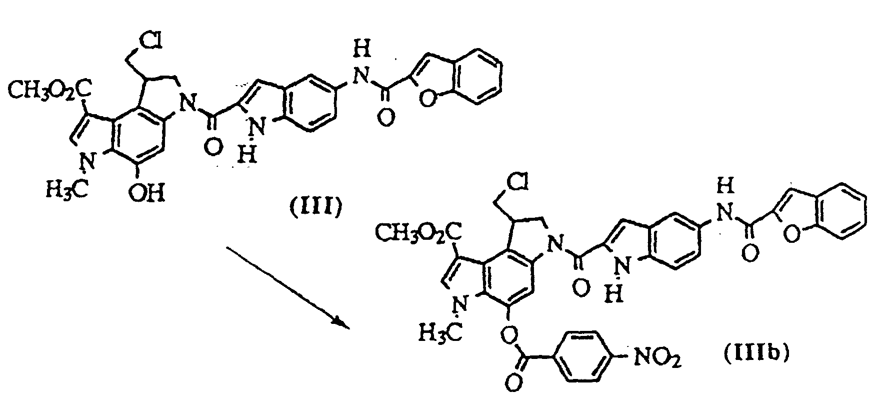

A solution of compound (I) (15 mg. 0.025 mmol), 4-nitrobenzoic acid (34 mg. 0.020 mmol) and N-z(3,3-dimethylaminopropyl)-N-ethylcarbodiimide hydrochloride (43 mg. 0.22 mmol) in dry dimethylformamide (1 mL) was kept at room temperature with agitation under argon for 20 hours.

The mixture resulting from adding brine (3mL) and hydrochloric acid (10%) (3 mL) was extracted with ethyl acetate (3X8 mL). The organic phase was washed with an aqueous saturated sodium bicarbonate solution (4 mL) and dried over anhydrous sodium sulfate. After concentrating a residue that was purified by chromatography in a silica flash gel column (12X1.5 cm ⌀) was obtained, eluting with CH₂Cl₂:EtOAc (4:1), yielding 15 mg (80%) of compound (IIIb).
m.p.: 260º C (dry, CH₂Cl₂), Rf.: 0.65 (hexane:acetone, 1:1).
¹H-NMR(Cl₃CD): 3.50 (dd, 1H, J=9,3 and 9.7 Hz, R₂CHCH₂NR₂), 3.86 (s, 3H, ArCH₃), 3.92 (s, 3H, ArCO₂CH₃), 4.00 (dd, 1H, J=11.0 and 13.7 Hz R₂CHCH₂NR₂), 4.65 (m, 1H, R₂CHCH₂ Cl), 4.71 (m, 1H, R₂CHCH₂Cl), 4.87 (d, 1H, J= 9.6 Hz, R₂CHCH₂Cl), 7.12 (s, 1H, ArH), 7.33 (t, 1H, J= 7.6 Hz, ArH), 7.47 (m, 3H, ArH), 7.58 (d, 1H, J=8.3 Hz, ArH), 7.61 (s, 1H, ArH), 7.72 (d, 1H, J= 7.6 Hz, ArH), 7.76 (s, 1H, ArH), 8,26 (s, 1H, ArH), 8.31 (s, 1H, ArH), 8.40 (s, 1H, ArNHCOAr), 8.44 (m, 4H, O₂NArH), 9.36 (s, 1H, ArNH).

### EXAMPLE OF BILOGICAL TESTS

### BIOLOGICAL ACTIVITY OF THE COMPOUNDS OF THE INVENTION

### Description of the P388 antitumoral model

The original tumoral line was chemically induced in 1955 in a DBA/2 mouse painting its skin with 3-methyl-cholanthrene. Normally, 1x10⁶ cells in ascitic fluid was implanted i.p. in CD₂F₁ mice. Treatment with the agent tested i.p. began one day after the tumor had been implanted and it is continued daily up to a total of 5 injections for synthetic products and of 9 injections for raw natural products. The results are expressed as percentage of the control survival time.

### Procedure

CDFl mice weighing 18-22 gr (+/- 3 gr) were implanted on day 0 of the test period o.1 ml. of a dilution of 1.0x10⁷ tumor cells coming from DBA/2N mice used for the propagation of the tumor.

On day 1 of the test period the animals were grouped at random in groups of six animals.Each group is weighed and the average weight is written down. Each compound to be tested in diluted in 4 levels of dosis for each multidose test as of the determination of the highest nontoxic dose (v.g. 400 mg/kg; 100 mg/kg; 10 mg/kg.)

Administration of the test compounds is started on day 1 using volumns of 1/2 drug injected i.p. Injections are given on days 1 to 9 of the test period, unless it is indicated to the contrary. The animals are weighed on day 5, which is the one which in this system is considered the day of toxicity. Toxicity in this test system is defined as:
A. 34 % deaths up to day 5 - acute toxicity
B. C/T<85% - chronic toxicity
C. A negative average of the weight change of the animals> 4 g up to day 5 - chronic toxicity

The data obtained upon testing the compounds in vivo in comparison with the P388 leukemic tumor model in mice are shown in the following table:

| Compound | Inject. dose (mg/kg) | Way | %C/T |
|---|---|---|---|
| (IV) | 0.300 | i.p. | 251.1** |
| (III) | 0.500 | i.p. | >391.3 |
| (VI) | 0.500 | i.p. | 210.9** |
| (V) | 0.050 | i.p. | 133.9** |
| Where %C/T means average survival of the tested group/average survival of the controls and untreated ones. | | | |

| | | | |
|---|---|---|---|
| * Significant activity (moderate): C/T > =125% ** Significant activity (strong): C/T > =175% | | | |

On the other hand, the results of the tests in vitro carried out on compounds (IIIa) and (IIIb) were the following:

| IC₅₀ ( µg/mL) | | | | |
|---|---|---|---|---|
| COMPOUND | P-388 | A-549 | HT-29 | MEL-28 |
| IIIa | 0.00001 | 0.00005 | 0.00025 | 0.00025 |
| IIIb | 0.00002 | 0.0001 | 0.0005 | 0.0005 |

## Claims

1. Pyrrolo [3,2-e] indole derivatives, **characterized in** having the following formulae (I), (Ia) and (II): 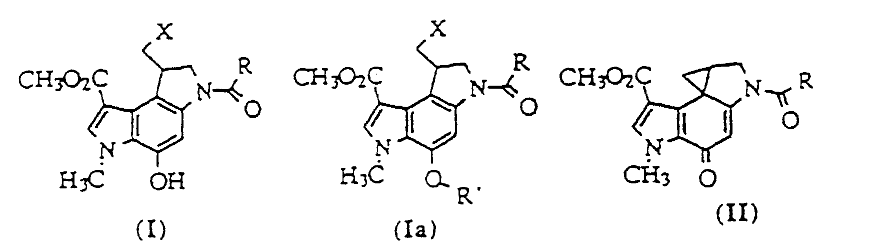 wherein R represents a phenyl, naphthyl, phenantryl or indolyl group, R' represents alkanoyl, alkenoyl, alkynoyl, aroyl or heteroaroyl group, and X represents chlorine, bromine or iodine.

2. Pyrrolo [3,2-e] indole derivatives according to claim 1, wherein R represents an indolyl group.

3. Pyrrolo [3,2-e] indole derivative having the following formula (III)

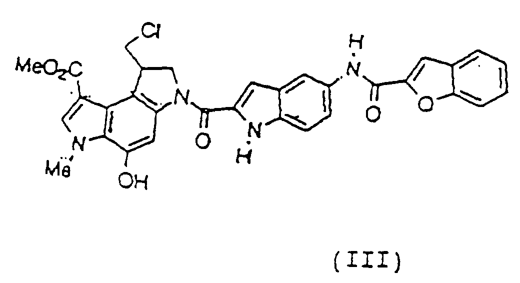

4. Pyrrolo [3,2-e] indole derivative having the following formula (IV)

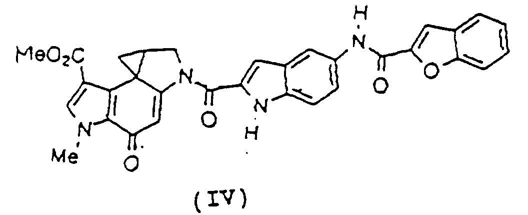

5. Pyrrolo [3,2-e] indole derivative having the following formula (V)

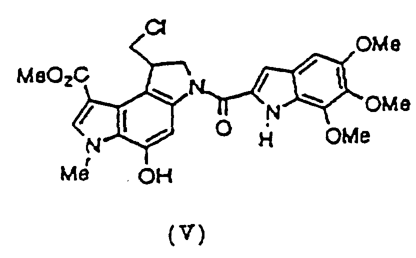

6. Pyrrolo [3,2-e] indole derivative having the following formula (VI)

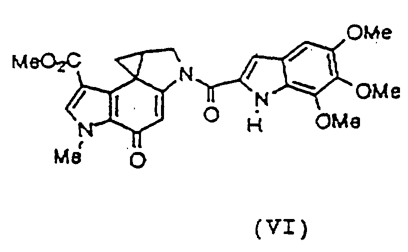

7. Pyrrolo [3,2-e] indole derivative having the following formula (IIIa)

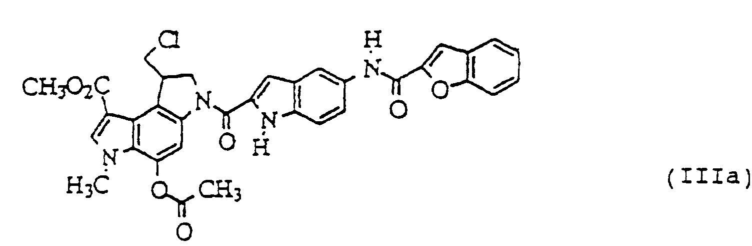

8. Pyrrolo [3,2-e] indole derivative having the following formula (IIIb)

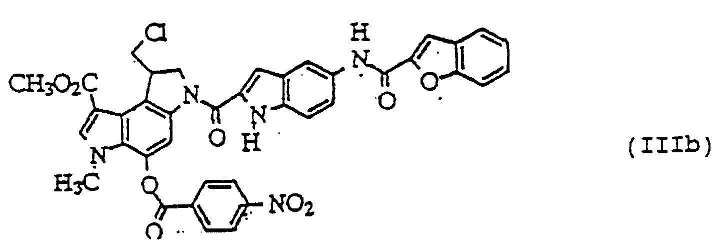

9. Process for the preparation of pyrrolo [3,2-e] indole derivatives according to any one of claims 1 to 8 comprising the steps:
a) subjecting to a deacylation reaction a compound of formula (VII): 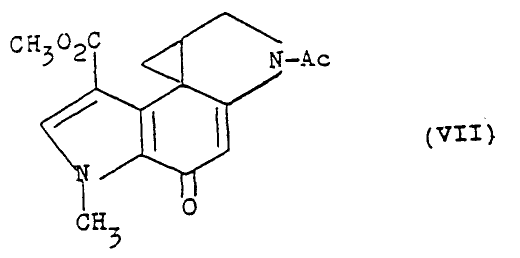 wherein Ac represents an acyl group, by treating the same with a base in an organic solvent, to produce a compound of formula (VIII): 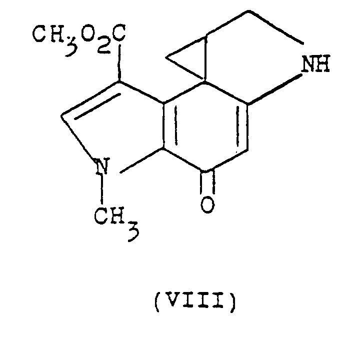
b) subjecting the compound (VIII) thus obtained to a reaction to open the cyclopropylring, by means of treating the same with an acid in an organic solvent, to produce the compound of formula (IX): 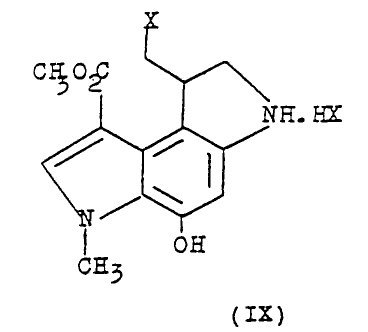 wherein X has the meaning given above for formula (I);
c) subjecting the compound (IX) thus obtained to a condensation reaction with an acid of formula
R-COOH (X)
wherein R has the meaning given above, or a reactive derivative thereof, to produce the active compound of formula (I) indicated above, carrying out said condensation in an organic solvent and in the presence of a condesing agent:
d) optionally, treating the compound of formula (I) thus obtained with a base in a suitable solvent to produce the compounds of formula (II):
e) optionally, treating the compounds of formula (I), obtained according to step c), alternatively with:
(a) a carboxylic acid in the presence of a condensing agent; or
(b) a carboxylic acid chloride in the presence of a base, or both alternatives in a suitable solvent;
to produce the the compounds of formula (la).

10. Pyrrolo [3,2-e] indole derivative according to any one of claims 1 to 8 for use in the treatment of cancer.

11. Use of a pyrrolo [3,2-e] indole derivative according to any one of claims 1 to 8 in the preparation of a medicament for the treatment of cancer.

## Patentansprüche

1. Pyrrolo[3,2-e]indolderivate, **dadurch gekennzeichnet, dass** sie die folgenden Formeln (I), (la) und (II) aufweisen: 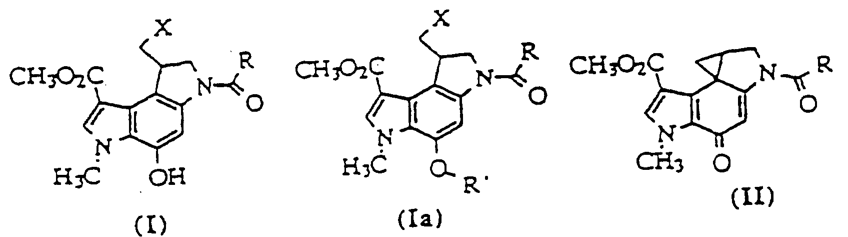 worin R eine Phenyl-, Naphthyl-, Phenanthryl- oder Indolylgruppe bedeutet, R' eine Alkanoyl-, Alkenoyl-, Alkinoyl-, Aroyl- oder Heteroaroylgruppe bedeutet und X Chlor, Brom oder lod bedeutet.

2. Pyrrolo[3,2-e]indolderivate nach Anspruch 1, worin R eine Indolylgruppe bedeutet.

3. Pyrrolo[3,2-e]indolderivat mit der folgenden Formel (III)

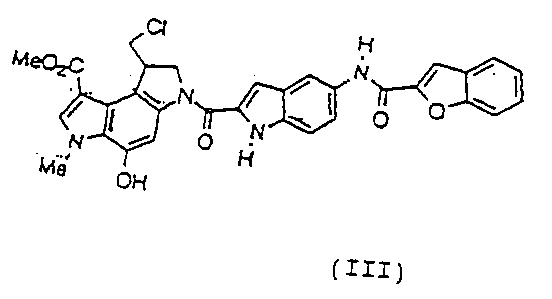

4. Pyrrolo[3,2-e]indolderivat mit der folgenden Formel (IV)

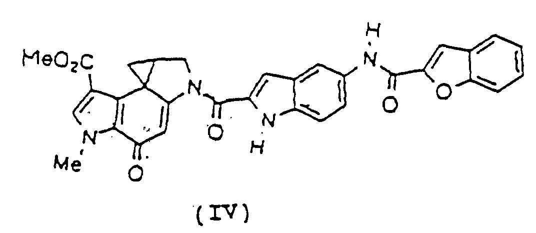

5. Pyrrolo[3,2-e]indolderivat mit der folgenden Formel (V)

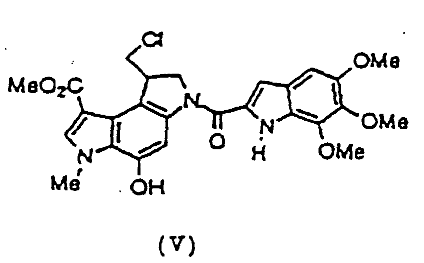

6. Pyrrolo[3,2-e]indolderivat mit der folgenden Formel (VI)

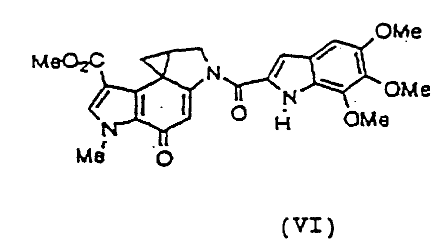

7. Pyrrolo[3,2-e]indolderivat mit der folgenden Formel (IIIa)

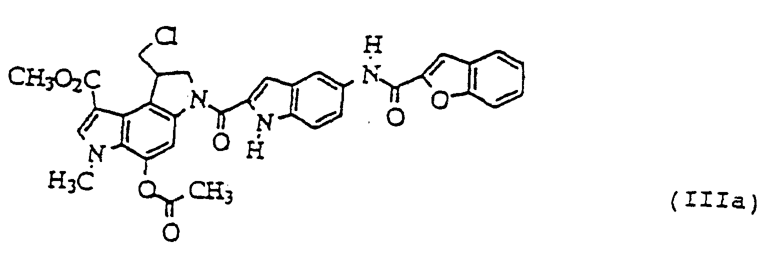

8. Pyrrolo[3,2-e]indolderivat mit der folgenden Formel (IIIb)

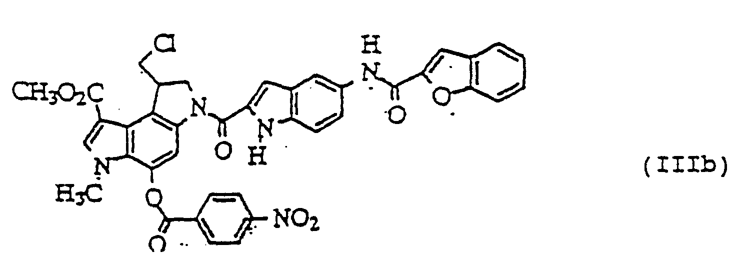

9. Verfahren zum Herstellen von Pyrrolo[3,2-e]indolderivaten nach einem der Ansprüche 1 bis 8, umfassend die Schritte:
a) Unterwerfen einer Verbindung der Formel (VII): 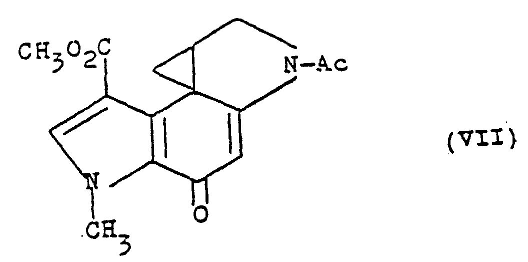 worin Ac eine Acylgruppe bedeutet, einer Desacylierungsreaktion durch Behandeln der Verbindung mit einer Base in einem organischen Lösungsmittel, um eine Verbindung der Formel (VIII) herzustellen: 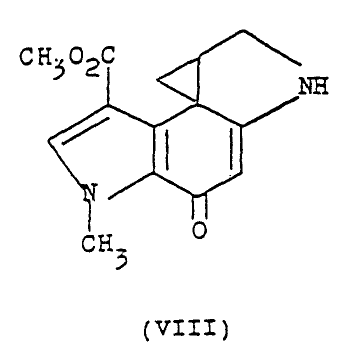
b) Unterwerfen der so erhaltenen Verbindung (VIII) einer Reaktion zum Öffnen des Cyclopropylrings durch Behandeln der Verbindung mit einer Säure in einem organischen Lösungsmittel, um die Verbindung der Formel (IX) herzustellen: 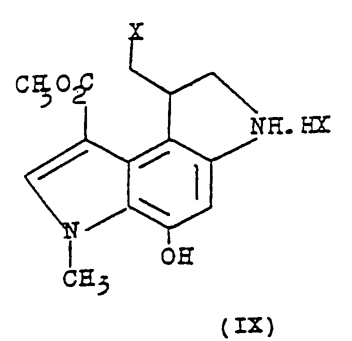 worin X die vorstehend für Formel (I) angegebene Bedeutung hat;
c) Unterwerfen der so erhaltenen Verbindung (IX) einer Kondensationsreaktion mit einer Säure der Formel
R-COOH (X)
worin R die vorstehend angegebene Bedeutung hat, oder mit einem reaktiven Derivat davon, um die aktive Verbindung der Formel (I) herzustellen, die vorstehend angegeben ist, wobei die Kondensation in einem organischen Lösungsmittel und in Gegenwart eines Kondensationsmittels durchgeführt wird:
d) gegebenenfalls Behandeln der so erhaltenen Verbindung der Formel (I) mit einer Base in einem geeigneten Lösungsmittel, um die Verbindungen der Formel (II) herzustellen:
e) gegebenenfalls Behandeln der gemäß Schritt c) erhaltenen Verbindungen der Formel (I) alternativ mit:
(a) einer Carbonsäure in Gegenwart eines Kondensationsmittels; oder
(b) einem Carbonsäurechlorid in Gegenwart einer Base, oder beiden Alternativen in einem geeigneten Lösungsmittel;
um die Verbindungen der Formel (la) herzustellen.

10. Pyrrolo[3,2-e]indolderivat nach einem der Ansprüche 1 bis 8 zur Verwendung bei der Behandlung von Krebs.

11. Verwendung eines Pyrrolo[3,2-e]indolderivats nach einem der Ansprüche 1 bis 8 bei der Herstellung eines Arzneimittels für die Behandlung von Krebs.

## Revendications

1. Dérivés de pyrrolo[3,2-e]indole, **caractérisés par** les formules (I), (Ia) et (II) suivantes : 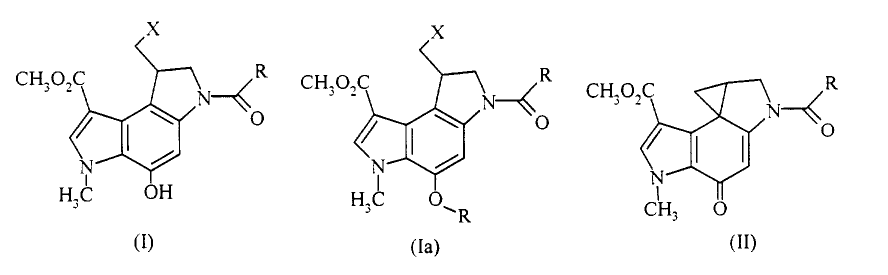 où R représente un groupe phényle, naphtyle, phénantryle ou indolyle, R' représente un groupe alcanoyle, alcénoyle, alkynoyle, aroyle ou hétéroaroyle et X représente le chlore, le brome ou l'iode.

2. Dérivés de pyrrolo[3,2-e]indole selon la revendication 1, où R représente un groupe indolyle.

3. Dérivé de pyrrolo[3,2-e]indole ayant la formule (III) suivante

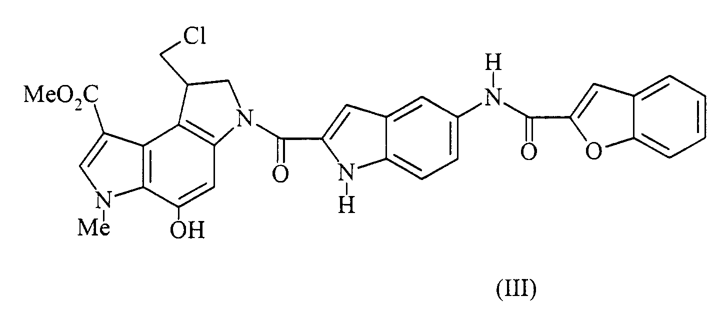

4. Dérivé de pyrrolo[3,2-e]indole ayant la formule (IV) suivante

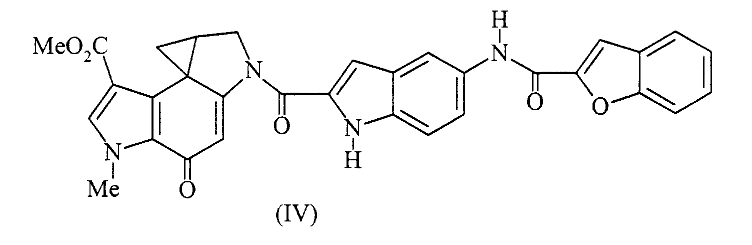

5. Dérivé de pyrrolo[3,2-e]indole ayant la formule (V) suivante

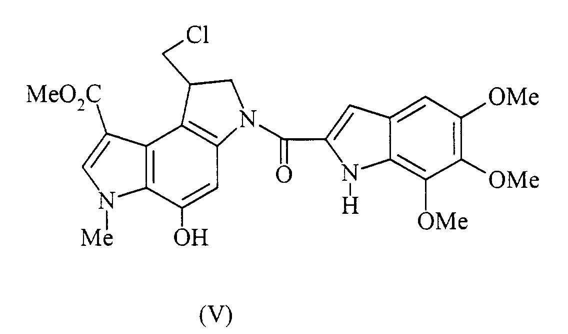

6. Dérivé de pyrrolo[3,2-e]indole ayant la formule (VI) suivante

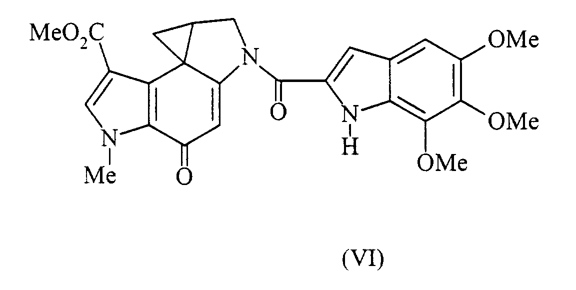

7. Dérivé de pyrrolo[3,2-e]indole ayant la formule (IIIa) suivante

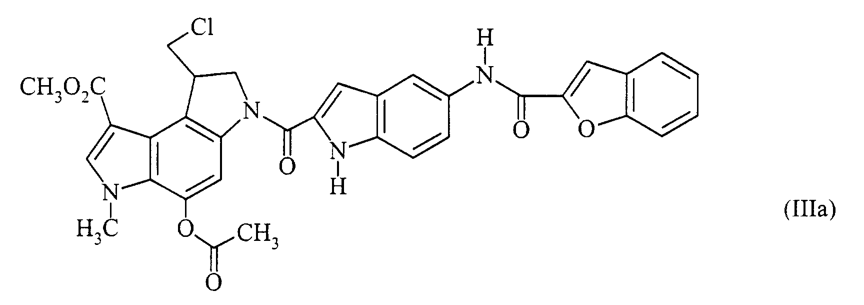

8. Dérivé de pyrrolo[3,2-e]indole ayant la formule (IIIb) suivante

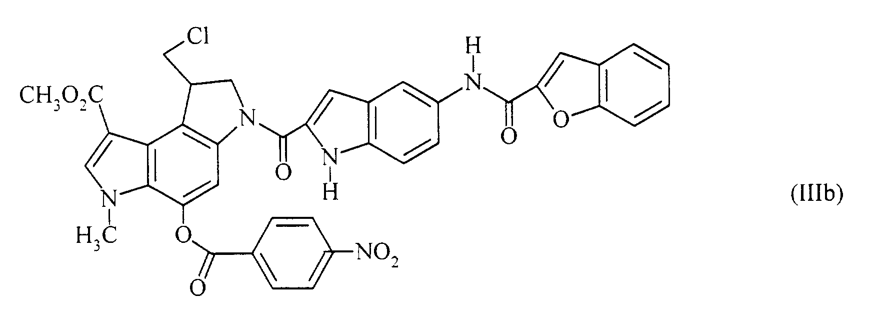

9. Procédé pour la préparation de dérivés de pyrrolo[3,2-e]indole selon l'une quelconque des revendications 1 à 8, comprenant les étapes consistant :
a) à soumettre à une réaction de déacylation un composé de la formule (VII): 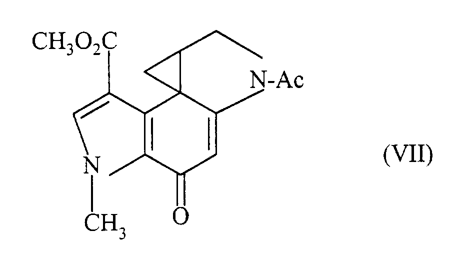 où Ac représente un groupe acyle par traitement de celui-ci avec une base dans un solvant organique, pour produire un composé de la formule (VIII) : 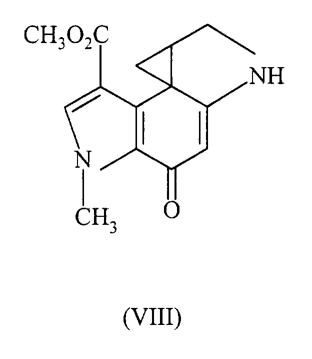
b) à soumettre le composé (VIII) ainsi obtenu à une réaction pour ouvrir le cycle cyclopropylex au moyen d'un traitement de celui-ci avec un acide dans un solvant organique pour produire le composé de la formule (IX): 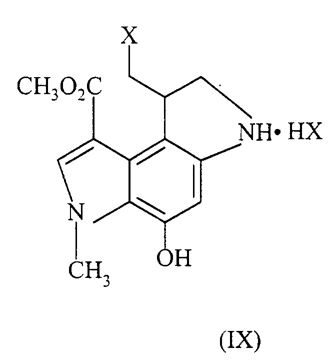 où X a la signification donnée ci-dessus pour la formule (I) ;
c) à soumettre le composé (IX) ainsi obtenu à une réaction de condensation avec un acide de la formule
R-COOH (X)
où R a la signification donnée ci-dessus, ou avec un dérivé réactif de celui-ci pour produire le composé actif de la formule (I) indiquée ci-dessus, à réaliser ladite condensation dans un solvant organique et en présence d'un agent de condensation ;
d) à traiter facultativement le composé de la formule (I) ainsi obtenu avec une base dans un solvant approprié pour produire les composés de la formule (II) ;
e) à traiter facultativement les composés de la formule (I) obtenus selon l'étape c) alternativement avec soit ;
(a) un acide carboxylique en présence d'un agent de condensation ; soit
(b) un chlorure d'acide carboxylique en présence d'une base, soit les deux alternatives dans un solvant approprié ;
pour produire les composés de la formule (Ia).

10. Dérivé de pyrrolo[3,2-e]indole selon l'une quelconque des revendications 1 à 8 pour une utilisation dans le traitement du cancer.

11. Utilisation d'un dérivé de pyrrolo[3,2-e]indole selon l'une quelconque des revendications 1 à 8 dans la préparation d'un médicament destiné au traitement du cancer.
